# EUROPÄISCHE PATENTANMELDUNG

(11) **EP 2 965 777 A1**
(43) Veröffentlichungstag der Anmeldung: **13.01.2016**
(21) Anmeldenummer: 15179171.2
(22) Anmeldetag: 29.12.2000
(51) Int. Cl.: A61M 16/06

(54) **VERFAHREN ZUR BEREITSTELLUNG, AUSBILDUNG ODER AUSWAHL EINER ATEMMASKE**

(30) Priorität: 11.01.2000 DE 10000790
(62) Teilanmeldung aus: 00128711.9
(71) Anmelder: ResMed R&D Germany GmbH, 82152 Martinsried (DE)
(72) Erfinder: Lang, Bernd, 82166 Gräfelfing (DE); Madaus, Dr. Stefan, 82166 Gräfelfing (DE)
(74) Vertreter: Vossius & Partner Patentanwälte Rechtsanwälte mbB

(57) **Zusammenfassung**

Verfahren zur Bereitstellung zur Ausbildung oder Auswahl einer Atemmaske für einen Maskenanwender geeigneter Angaben, wobei das Verfahren aufweist: Erzeugen eines Raumdatensatzes durch Einlesen der individuellen räumlichen Gesichtskontur des Maskenanwenders zumindest im Nasen- und/oder Mundbereich, wobei der Raumdatensatz durch einen optischen Abtastvorgang und/oder eine stereofotografische Messung erzeugt wird.

## Beschreibung

Die Erfindung betrifft eine Atemmaskenanordnung zur Zufuhr eines Atemgases zu einem Maskenanwender unter Überdruck sowie ein Verfahren zur Bereitstellung einer derartigen Atemmaskenanordnung.

Atemmaskenanordnungen der eingangs genannten Art werden allgemein dazu verwendet, einer Person ein Atemgas unter einem vorbestimmten Überdruck zuzuführen und finden insbesondere während des Aufenthalts von Personen in einer belasteten Atmosphäre, beispielsweise im Untertagebau, in Lackierereien, im industriellen Bereich, sowie insbesondere auch im medizinischen Bereich Anwendung. Häufig werden derartige Atemmasken über einen vergleichsweise langen Zeitraum von mehreren Stunden getragen. Der Dichtigkeit der Atemmasken sowie einem hinreichenden Tragekomfort kommt hierbei besondere Bedeutung zu. Um einen möglichst hohen Tragekomfort zu gewährleisten, ist man dazu übergegangen, die unmittelbar auf den Gesichtsbereich des Atemmaskenanwenders aufliegenden Dichtlippen dünnwandig zu gestalten. Derartige Dichtlippen können entweder einstückig mit einem Maskenbasiskörper ausgebildet sein, oder an einen - vorzugsweise aus einem transparenten Material gefertigten - hartschalenartigen Maskenbasiskörper angesetzt sein. Es hat sich gezeigt, daß sich in vielen Fällen die Bereitstellung eines für den jeweiligen Maskenanwender passenden Atemmaskensystems als vergleichsweise schwierig erweist, da Atemmaskensysteme, die zunächst hinsichtlich einer ausreichenden Dichtigkeit als überzeugend erscheinen, nach längerer Zeit in Bezug auf den Tragekomfort beanstandet werden.

Unter dem Eindruck dieses Problems liegt der Erfindung die Aufgabe zugrunde, ein Verfahren zu schaffen, durch welches eine Atemmaskenanordnung in der für den jeweiligen Maskenanwender günstigsten Konfiguration bereitgestellt werden kann.

Diese Aufgabe wird erfindungsgemäß durch ein Verfahren gelöst, bei welchem zur Ausbildung Zusammenstellung oder Auswahl der Atemmaskenanordnung geeignete Angaben auf Grundlage eines Raumdatensatzes erzeugt werden, wobei der Raumdatensatz durch Einlesen der individuellen räumlichen Gesichtskontur des Maskenanwenders zumindest im Nasen und/oder Mundbereich desselben erzeugt wird.

Dadurch wird es auf vorteilhafte Weise möglich, auf objektive Weise das für die individuelle Gesichtskrümmung des Maskenanwenders günstigste Maskensystem bereitzustellen. Im Rahmen eines vorzugsweise durch ein Datenverarbeitungssystem durchgeführten Auswahlvorganges kann hierbei überprüft werden, ob sich eine hinreichende Dichtwirkung mit bestimmten vorhandenen Atemmaskensystemen erreichen läßt, oder ob Bedarf nach einer besonders auf den Maskenanwender abgestimmten Dichtungsstruktur besteht.

Die Auswertung oder Bewertung der erfaßten Raumdaten des Gesichtsbereiches des Maskenanwenders kann in vorteilhafter Weise über ein externes Rechnersystem erfolgen auf welches beispielsweise über das Internet Zugriff genommen werden kann. Auf diesem externen Rechnersystem können dann FEM-Berechnungsverfahren durchgeführt werden über welche beispielsweise die Verformung der Atemmaske durch den Atemmaskenanwender simuliert und die sich hierbei ergebende Flächenpressung beschrieben wird.

Insbesondere für den Fall, daß der Raumdatensatz eine besonders eigentümliche Gesichtskrümmung im Umgebungsbereich der Nase beschreibt, wird gem. einer besonders bevorzugten Ausführungsform des Verfahrens vorgeschlagen das Atemmaskensystem unter Verwendung modifizierter Komponenten zusammenzustellen. In vorteilhafter Weise wird hierbei der Dichtlippenabschnitt der Atemmaske derart ausgebildet insbes. modifiziert, daß dessen räumlicher Verlauf auf die individuelle Gesichtskrümmung im potentiellen Dichtlippenauflagebereich abgestimmt ist. Falls anhand des erfaßten Raumdatensatzes erkennbar ist, daß der individuellen Gesichtskrümmung des Maskenanwenders voraussichtlich durch eine bestimmte, vorhandene Dichtungsstruktur Rechnung getragen werden kann, ist es auch möglich, auf Grundlage des erfaßten Raumdatensatzes ein Dichtlippenelement, oder vorzugsweise auch einen zugehörigen Maskenbasiskörper aus einem vorgefertigten Sortiment automatisch auszuwählen.

Der Raumdatensatz wird gemäß einer besonders bevorzugten Ausführungsform in digitaler Form gespeichert. Auf Grundlage dieses gespeicherten Raumdatensatzes kann dann ein bevorzugter Verlauf einer Gesichtsabdichtungszone ermittelt werden. Es ist auch möglich, anhand dieses Raumdatensatzes zu überprüfen, welche Gesichtsauflagezonen sich bei Verwendung der verfügbaren Atemmasken ergeben würden. Auf diese Weise kann unmittelbar über ein Datenverarbeitungssystem, vorzugsweise unter visueller Unterstützung, überprüft werden, welches Atemmaskensystem sich für den Anwender als besonders vorteilhaft erweist. Auf mühsame und zeitintensive Anproben sowie auf das in diesem Zusammenhang üblicherweise erforderliche Reinigen der erprobten Maskensysteme kann hierbei auf vorteilhafte Weise verzichtet werden.

Gemäß einer besonders bevorzugten Ausführungsform der Erfindung umfaßt der Raumdatensatz auch noch Raumdaten, die den Stirnbereich des Maskenanwenders mit beschreiben. Hierdurch wird es auf vorteilhafte Weise möglich, auch gleichzeitig die optimalen Befestigungsstrukturen, insbesondere ein passendes Stirnhalterelement, für die Atemmaske zu ermitteln.

Insbesondere bei einer mehrteiligen Ausführungsform des Atemmaskensystems, d.h., insbesondere bei der Verwendung einer Atemmaske mit einer aus einem Elastomermaterial gebildeten Dichtlippenstruktur und einer aus einem transparenten Hartschalenwerkstoff gebildeten Maskenbasiskörper wird das Maskensystem derart ausgewählt, daß sich zwischen der Innenwandung des Maskenkörpers und dem Nasenrückenbereich des Maskenanwenders ein im Hinblick auf die Atemgaszuströmung optimaler Zwischenraum ergibt.

Durch das erfindungsgemäße Verfahren wird es auf vorteilhafte Weise möglich, aus einer Vielzahl unterschiedlichster, vorzugsweise vorgefertigter Dichtlippenteile das jeweils günstigste Dichtlippenteil sowie einen geeigneten Maskenkörper auszuwählen. Es ist auch möglich, das Dichtlippenteil auf Grundlage des Raumdatensatzes zu modifizieren. Hierbei ist es beispielsweise möglich, das Dichtlippenteil mit einer plastisch verformbaren Verstärkungseinlage zu versehen, die auf Grundlage des eingelesenen Raumdatensatzes bedarfsgerecht verformt wird. Vorzugsweise ist hierzu eine Stützstruktur vorgesehen, die das Dichtungsteil in eine der individuellen Gesichtskrümmung im Bereich der Gesichtsauflagefläche Rechnung tragende Konfiguration zwängt. Es ist auch möglich, die optimale Kontur der Nasendurchtrittsöffnung zu ermitteln und das Dichtlippenteil ggf. entsprechend zu modifizieren.

Alternativ hierzu oder auch in Kombination mit diesen Maßnahmen ist es auch möglich, insbesondere bei einer zweiteiligen Atemmaske den Verbindungsbereich zwischen dem Dichtungsteil, bzw. dem Dichtlippenteil, und der Hartschale, d.h. dem Maskenbasiskörper, auf Grundlage des Raumdatensatzes auszubilden. Beispielsweise ist es hierzu möglich, eine entsprechende umlaufende Nut über ein bahngesteuertes Werkzeug in den Maskenkörper einzuformen, so daß das Dichtungsteil entsprechend dem Verlauf der Nut vorverformt wird.

Es hat sich gezeigt, daß der Raumdatensatz in vorteilhafter Weise mit einer Auflösung erzeugt wird, die die Krümmung des Gesichts des Maskenanwenders wenigstens mit einer Auflösung von 16 Meßpunkten je Quadratzentimeter beschreibt. Es ist möglich, eine feinere Abtastung vorzunehmen, oder, die vergleichsweise grobe Abtastung der individuellen Gesichtskontur durch Interpolation der erfaßten Koordinaten noch weiter zu verfeinern.

Eine besonders präzise Erfassung der individuellen Gesichtskontur des Maskenanwenders wird gem. einer besonders bevorzugten Ausführungsform der Erfindung durch eine stereofotografische Messung erreicht. Alternativ dazu oder auch in Kombination mit dieser stereofotografischen Messung ist es auch möglich, den Raumdatensatz durch einen optischen, insbesondere laseroptischen Abtastvorgang zu erzeugen.

In besonders vorteilhafter Weise wird der Raumdatensatz dadurch erzeugt, daß mittels einer Digitalkamera das Gesicht des Maskenanwenders fotografiert wird, wobei auf das Gesicht des Maskenanwenders aus einer vorzugsweise von der optischen Achse der Kamera abweichenden Richtung ein Muster, beispielsweise ein Quadratraster projiziert wird. Der so über die Digitalkamera aufgenommene Bilddatensatz kann anhand eines Bildverarbeitungsprogramms ausgewertet werden zur Erzeugung des zur Auswahl oder Ausbildung des Atemmaskensystems geeigneten Raumdatensatzes. Vorzugsweise wird hierbei das gesamte Gesicht des Maskenanwenders fotografiert und wenigstens im Umgebungsbereich der Nase und vorzugsweise auch im Stirnbereich des Maskenanwenders eine entsprechende Generierung von Raumkoordinaten vorgenommen. Hierdurch wird es möglich, unmittelbar an einem PC eine Überprüfung der vorhandenen Atemmaskensysteme vorzunehmen.

Hinsichtlich einer Atemmaske wird die eingangs angegebene Aufgabe auch durch eine Atemmaske gelöst mit einem Maskenkörper und einem aus einem elastischen Material gebildeten Dichtlippenteil, das beim Aufsetzen der Atemmaske auf das Gesicht eines Maskenanwenders einen vom Maskenkörper begrenzten Maskeninnenbereich gegenüber der Umgebung abdichtet, wobei der räumliche Verlauf des Dichtlippenteils nach Maßgabe eines Raumdatensatzes ausgebildet oder ausgewählt ist, der durch Abtasten der individuellen räumlichen Gesichtskrümmung des Maskenanwenders generiert ist.

Vorzugsweise ist der räumliche Verlauf des Dichtlippenteils hierbei durch ein Stützstrukturteil bestimmt, das nach Maßgabe des Raumdatensatzes ausgebildet oder zumindest ausgewählt ist. Eine besonders robuste Ausführungsform dieses Stützstrukturteils ist gem. einer bevorzugten Ausführungsform der Erfindung dadurch gegeben, daß das Stützstrukturteil durch ein Bügelelement gebildet ist. Es ist auch möglich, mehrere Stützstrukturteile vorzusehen, um der individuellen Gesichtskrümmung des Maskenanwenders noch besser Rechnung tragen zu können.

Gemäß einer besonders bevorzugten Ausführungsform wird das Dichtlippenteil derart geformt ausgebildet, insbesondere durch das Stützstrukturteil derart deformiert, daß sich bei aufgesetztem Maskenelement eine vorgegebene Kontaktdruckverteilung ergibt. Diese Kontaktdruckverteilung kann hierbei derart gewählt sein, daß sich im Bereich tragfähigerer Gesichtszonen ein höherer Kontaktdruck ergibt, wogegen sich im Bereich von druckempfindlichen Gesichtszonen, insbesondere im oberen Nasenrückenbereich nur vergleichsweise geringe Anpreßdrücke ergeben.

Das Dichtlippenteil ist gem. einer besonders bevorzugten Ausführungsform der Erfindung von dem Stützstrukturbauteil lösbar ausgebildet. Dadurch wird es möglich, das Dichtlippenteil ggf. auszuwechseln und das entsprechend individuell vorgeformte Stützstrukturbauteil an ein neues Dichtlippenteil anzusetzen, wodurch dieses wieder die gewünschte individuelle Gestalt erhält.
1. Verfahren zur Bereitstellung zur Ausbildung oder Auswahl einer Atemmaske für einen Maskenanwender geeigneter Angaben gekennzeichnet durch den Schritt des Erzeugens eines Raumdatensatzes durch Einlesen der individuellen räumlichen Gesichtskontur des Maskenanwenders zumindest im Nasen- und/oder Mundbereich.
2. Verfahren nach Aspekt 1, dadurch gekennzeichnet, daß auf Grundlage des erfaßten Raumdatensatzes ein Dichtlippenabschnitt der Atemmaske derart ausgebildet wird, daß dessen räumlicher Verlauf auf die individuelle Gesichtskrümmung im Dichtlippenauflagebereich abgestimmt ist.
3. Verfahren nach Aspekt 1, dadurch gekennzeichnet, daß auf Grundlage des erfaßten Raumdatensatzes ein Dichtlippenteil und/oder ein Maskenbasiskörper und/oder ein Stirnauflageelement ausgewählt wird.
4. Verfahren nach wenigstens einem der Aspekte 1 bis 3, dadurch gekennzeichnet, daß der Raumdatensatz in digitaler Form gespeichert wird, und daß auf Grundlage dieses Datensatzes ein geeigneter Verlauf der Gesichtsabdichtungszone ermittelt wird.
5. Verfahren nach wenigstens einem der Aspekte 1 bis 4, dadurch gekennzeichnet, daß auf Grundlage des erfaßten Raumdatensatzes wenigstens ein Maskenkörper aus einer Serie von Maskenkörpern derart ausgewählt wird, daß zwischen der Innenwandung des Maskenkörpers und dem Nasenbereich des Maskenanwenders ein hinreichender Zwischenraum verbleibt.
6. Verfahren nach wenigstens einem der Aspekte 1 bis 5, dadurch gekennzeichnet, daß auf Grundlage des erfaßten Raumdatensatzes wenigstens ein geeignetes Dichtlippenteil aus einer Serie von Dichtlippenteile ausgewählt wird.
7. Verfahren nach wenigstens einem der Aspekte 1 bis 6, dadurch gekennzeichnet, daß das Dichtlippenteil auf Grundlage des Raumdatensatzes modifiziert ausgebildet wird.
8. Verfahren nach wenigstens einem der Aspekte 1 bis 7, dadurch gekennzeichnet, daß eine dem Dichtlippenteil zugeordnete Stützstruktur nach Maßgabe des Raumdatensatzes derart ausgebildet wird, daß die Stützstruktur das Dichtlippenteil in eine der individuellen Gesichtskrümmung im Bereich der Gesichtsauflagefläche Rechnung tragende Konfiguration zwängt.
9. Verfahren nach wenigstens einem der Aspekte 1 bis 8, dadurch gekennzeichnet, dadurch gekennzeichnet, daß ein Verbindungsbereich zwischen dem Dichtlippenteil und dem Maskenkörper auf Grundlage des Raumdatensatzes ausgebildet wird.
10. Verfahren nach wenigstens einem der Aspekte 1 bis 9, dadurch gekennzeichnet, daß der Raumdatensatz derart erzeugt wird, daß dieser die Krümmung des Gesichts zumindest abschnittsweise mit einer Auflösung von wenigstens 16 Meßpunkten je cm² wiedergibt.
11. Verfahren nach wenigstens einem der Aspekte 1 bis 10, dadurch gekennzeichnet, daß durch Interpolation der erfaßten Koordinaten die Auflösung noch weiter verfeinert wird.
12. Verfahren nach wenigstens einem der Aspekte 1 bis 11, dadurch gekennzeichnet, daß der Raumdatensatz durch stereofotografische Messung erzeugt wird.
13. Verfahren nach wenigstens einem der Aspekte 1 bis 11, dadurch gekennzeichnet, daß der Raumdatensatz durch einen optischen insbesondere laseroptischen Abtastvorgang erzeugt wird.
14. Verfahren nach wenigstens einem der Aspekte 1 bis 13, dadurch gekennzeichnet, daß der Raumdatensatz dadurch erzeugt wird, daß mittels einer Digitalkamera das Gesicht des Maskenanwenders photographiert wird, wobei auf das Gesicht aus einer vorzugsweise von der optischen Achse der Kamera abweichenden Richtung ein Muster projiziert wird.
15. Atemmaskenanordnung mit einem Maskenkörper und einem aus einem elastischen Material gebildeten Dichtlippenteil das beim Aufsetzen der Atemmaske auf das Gesicht eines Maskenanwenders einen vom Maskenkörper begrenzten Maskeninnenbereich gegenüber der Umgebung abdichtet, dadurch gekennzeichnet, daß das Dichtlippenteil nach Maßgabe eines Raumdatensatzes ausgebildet oder ausgewählt ist, der durch Abtasten der individuellen räumlichen Gesichtskrümmung des Maskenanwenders generiert ist.
16. Atemmaskenanordnung nach Aspekt 15, dadurch gekennzeichnet, daß der räumliche Verlauf des Dichtlippenteils durch ein Stützstrukturteil bestimmt ist das nach Maßgabe des Raumdatensatzes ausgebildet oder ausgewählt ist.
17. Atemmaskenanordnung nach Aspekt 15 oder 16, dadurch gekennzeichnet, daß das Stützstrukturteil durch ein Bügelelement gebildet ist.
18. Atemmaskenanordnung nach wenigstens einem der Aspekte 15 bis 17, dadurch gekennzeichnet, daß mehrere Stützstrukturteile vorgesehen sind.
19. Atemmaskenanordnung nach wenigstens einem der Aspekte 15 bis 18, dadurch gekennzeichnet, daß das Stützstrukturteil das Dichtlippenteil derart verformt, daß sich bei aufgesetztem Maskenelement eine vorgegebene Kontaktdruckverteilung ergibt.
20. Atemmaskenanordnung nach wenigstens einem der Aspekte 15 bis 19, dadurch gekennzeichnet, daß das Dichtlippenteil von dem Stützstrukturbauteil lösbar ist.

Weitere Einzelheiten der Erfindung ergeben sich aus der nachfolgenden Beschreibung in Verbindung mit der Zeichnung.

Es zeigen:
**Fig. 1** das Gesicht eines Maskenanwenders, wobei der für die Auswahl, bzw. Ausbildung des Atemmaskensystems relevante Gesichtsbereich hier mit Strichpunktlinien umrahmt ist;
**Fig. 2** eine vereinfachte perspektivische Ansicht eines auf Grundlage der individuellen Gesichtskrümmung des Maskenanwenders erfaßten räumlichen Verlaufs eines Dichtlippenauflagebereiches;
**Fig. 3** eine vereinfachte Skizze zur Erläuterung einer bevorzugten Kontaktdruckverteilung in dem Dichtlippenauflagebereich des Atemmaskensystems;
**Fig. 4** eine vereinfachte perspektivische Ansicht eines hier durch einen gebogenen Stahlbügel gebildeten Stützstrukturbauteils, das ein Dichtlippenelement in eine auf die individuelle Gesichtskrümmung des Atemmaskenanwenders abgestimmte Gestalt verformt;
**Fig. 5a** eine vereinfachte Schnittansicht zur Erläuterung eines bevorzugten Aufbaus eines Dichtlippenelementes einer Atemmaske, das durch ein Stützstrukturteil (Fig. 4) in eine bestimmte Gestalt vorgeformt ist;
**Fig. 5b** eine vereinfachte Schnittansicht zur Erläuterung einer Befestigungsrandstruktur zwischen einem Dichtlippenteil und dem Randabschnitt einer Maskenkörper-Hartschale;
**Fig. 5c** eine vereinfachte Schnittansicht durch die unmittelbar auf der Gesichtsfläche des Atemmaskenanwenders aufliegenden Dichtlippe mit darin eingebetteten plastisch verformbaren Drahteinlagen; und
**Fig. 6** ein vereinfachtes Flußdiagramm für einen bevorzugten Ablauf des erfindungsgemäßen Verfahrens.

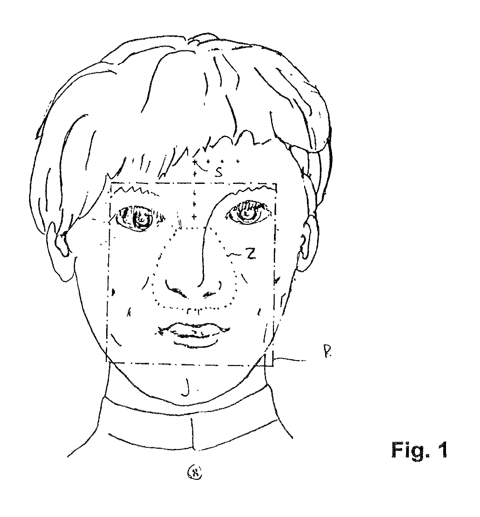

In Fig. 1 ist vereinfacht das Gesicht eines Atemmaskenanwenders dargestellt, wobei die individuelle räumliche Gesichtskrümmung des Maskenanwenders wenigstens in dem hier durch den in Strichpunktlinien angedeuteten Rahmen R erfaßt wird. Die Erfassung dieses Raumdatensatzes erfolgt vorzugsweise mittels einer Digitalkamera. Diese Digitalkamera kann vorzugsweise als stereofotografische Kamera ausgebildet sein. Die Ermittlung der Raumkoordinaten anhand des durch die Kamera aufgezeichneten Bildes kann dadurch unterstützt werden, daß auf das Gesicht des Maskenanwenders zusätzlich ein Muster in Form von Punkten oder vorzugsweise Gitterlinien projiziert wird. Anhand der derart gewonnenen, hinsichtlich der individuellen Gesichtskrümmung des Maskenanwenders indikativen Daten kann anhand eines Computerprogramms ein optimaler Verlauf einer Dichtlippen-Auflagezone Z, die hier durch Punkte angedeutet ist, ermittelt werden. Vorzugsweise wird auch der obere Nasenrückenbereich sowie der Stirnbereich des Maskenanwenders vermessen. Hierdurch wird es möglich, ein vollständiges Atemmaskensystem zusammenzustellen, das der individuellen Gesichtskrümmung des Maskenanwenders optimal Rechnung trägt.

Die individuelle Gesichtskrümmung des Maskenanwenders wird vorzugsweise durch Polarkoordinaten oder auch alternativ hierzu durch karthesische Koordinaten beschrieben. Die Auflösung der Abtastung der individuellen Gesichtskrümmung liegt zumindest im unmittelbaren Umgebungsbereich der Nase und insbesondere im Bereich des Nasenrückens bei wenigstens 16 Meßpunkten je Quadratzentimeter.

Im Stirnbereich des Maskenanwenders kann die Auflösung ggf. gröber gewählt werden oder auf ausgewählte Meßorte beschränkt sein.

Die so über ein Meßsystem erfaßten Raumdaten können unmittelbar auf ein Computersystem übertragen und hier visualisiert werden. Anhand dieser visualisierten Gesichtsdaten kann ggf. vollständig automatisiert eine Vorauswahl aus vorhandenen Maskensystemen getroffen werden. Die vorhandenen Maskensysteme können auf ihre Paßform überprüft werden. Hierbei wird es möglich, vorzugsweise über ein FEM-Berechnungsverfahren die zu erwartende Gesichtsflächenpressung zu errechnen und auf ggf. unzulässig hohe Flächenpressungen zu überprüfen. Auch ist es möglich, Zonen von vergleichsweise geringer Flächenpressung zu ermitteln und dadurch potentielle Undichtigkeitsstellen zu erkennen.

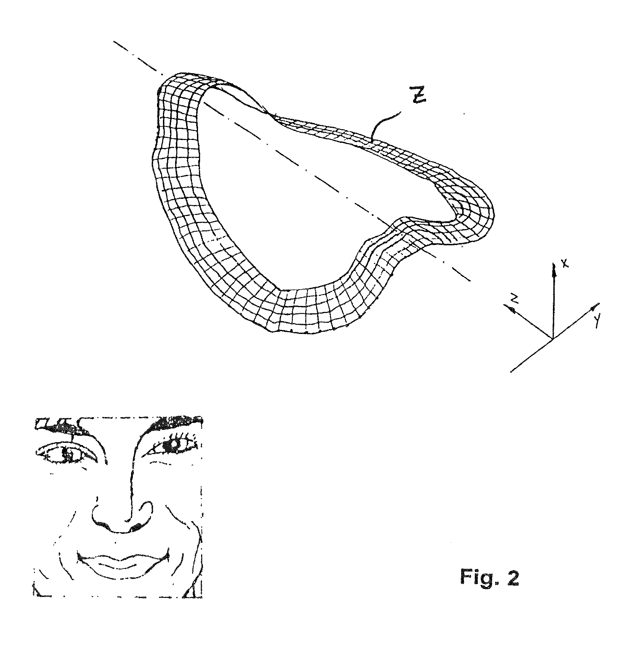

In Fig. 2 ist die Fig. 1 durch den Buchstaben z angedeutete Maskenauflagefläche dargestellt. Anhand dieser Maskenauflagefläche kann nochmals im einzelnen das Abdichtungsverhalten einer ausgewählten Dichtlippenstruktur überprüft werden.

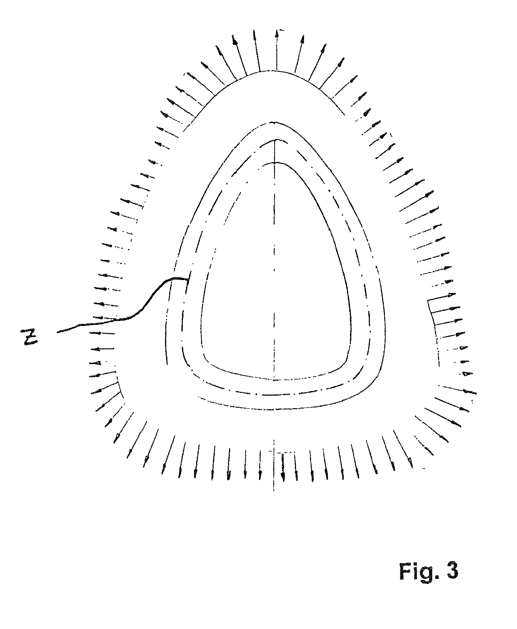

In Fig. 3 ist die für die Maskenauflagezone z gem. Fig. 2 optimale Anpreßdruckverteilung dargestellt. Wie hier deutlich erkennbar ist insbesondere im Bereich der seitlichen Flanken des Nasenrückens sowie im unteren Oberlippenbereich die zulässige Flächenpressung deutlich abgesenkt. Anhand dieser Sollflächenpressung kann überprüft werden, ob innerhalb eines vertretbaren Toleranzbereiches diese Sollflächenpressung mit vorhandenen Dichtlippensystemen erreicht werden kann, oder ob es einer besonderen Modifikation, bzw. Ausgestaltung eines Dichtlippenelementes bedarf. Sofern festgestellt wird, daß diese gewünschte Anpreßdruckverteilung mit vorhandenen Dichtlippenelementen nicht ohne weiteres erreicht werden kann, ist es möglich, das Dichtlippenelement beispielsweise durch ein Stützstrukturteil bedarfsgerecht zu deformieren.

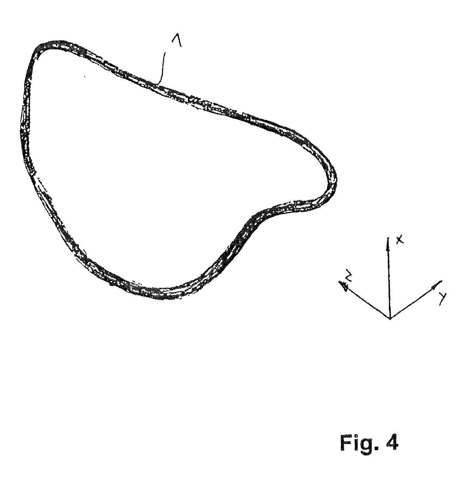

Ein derartiges Stützstrukturteil 1 ist beispielhaft in Fig. 4 dargestellt. Dieses Stützstrukturteil ist auf Grundlage des eingelesenen Raumdatensatzes räumlich gekrümmt ausgebildet und zwängt ein Standard-Dichtlippenteil in eine vorgegebene Form, wodurch die in Fig. 3 angegebene vorteilhafte Anpreßdruckverteilung erreicht werden kann.

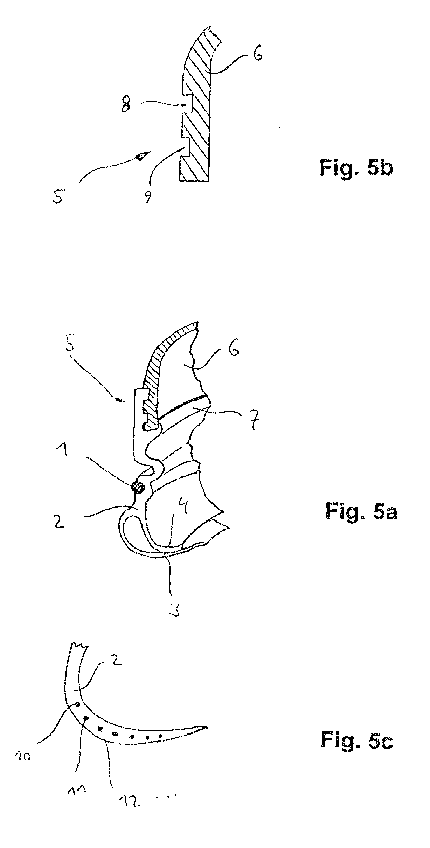

In Fig. 5a ist vereinfacht ein bevorzugter Querschnitt eines Dichtlippenbauteiles 2 mit einem darin eingesetzten Stützstrukturteil dargestellt. Das hier dargestellte Dichtlippenteil 2 umfaßt eine dünne unmittelbar zur Auflage auf der Gesichtsfläche des Maskenanwenders vorgesehene Außendichtlippe 3 sowie eine zweite Innendichtlippe 4, die abschnittsweise auf der Außendichtlippe 3 aufsitzt. Das Dichtlippenteil 2 ist über eine Umfangsnutenstruktur 5 mit einer transparenten Hartschale 6 eines Maskenbasiskörpers verbunden. Im inneren Übergangsbereich zwischen dem Dichtlippenteil 2 und dem Hartschalenteil 6 ist eine Umfangswulststruktur 7 vorgesehen, die abdichtend an der Innenfläche der Hartschale des Maskenkörpers anliegt.

In Fig. 5b ist nochmals der hartschalenkörperseitige Teil der Verbindungsstruktur 5 dargestellt. Die hier vorgesehenen Nuten 8, 9 können ggf. unter Verwendung eines bahngesteuerten Werkzeuges, insbesondere Fräsers, derart individuell in den Hartschalenkörper 6 eingefräst werden, daß sich bereits aus der Ankoppelung des Dichtlippenteiles 2 an den Hartschalenkörper 6 eine auf die individuelle Gesichtskrümmung des Maskenanwenders abgestimmte Vorverformung des Dichtlippenelementes 2 ergibt.

In Fig. 5c ist eine weitere Möglichkeit dargestellt, durch welche der räumliche Verlauf einer zur Auflage auf dem Gesicht des Maskenanwenders vorgesehenen Dichtlippe individuell dieser Gesichtskrümmung angepaßt werden kann. Bei der hier dargestellten Ausführungsform sind in das Elastomermaterial des Dichtlippenelementes eine Vielzahl feiner Draht-elemente 10, 11, 12 eingebettet, die bedarfsweise plastisch verformt werden können und hierbei dem Dichtlippenelement 2 eine vorgegebene Gestalt verleihen. Die Verformung dieser Einlagen kann ggf. unter Verwendung eines entsprechend gesteuerten Biegewerkzeuges erfolgen. Es ist auch möglich, das Dichtlippenelement ggf. manuell zu verformen und das manuell verformte Dichtlippenelement zu vermessen und mit dem für den jeweiligen Maskenanwender erzeugten Raumdatensatz unter entsprechenden Paßformkriterien zu überprüfen.

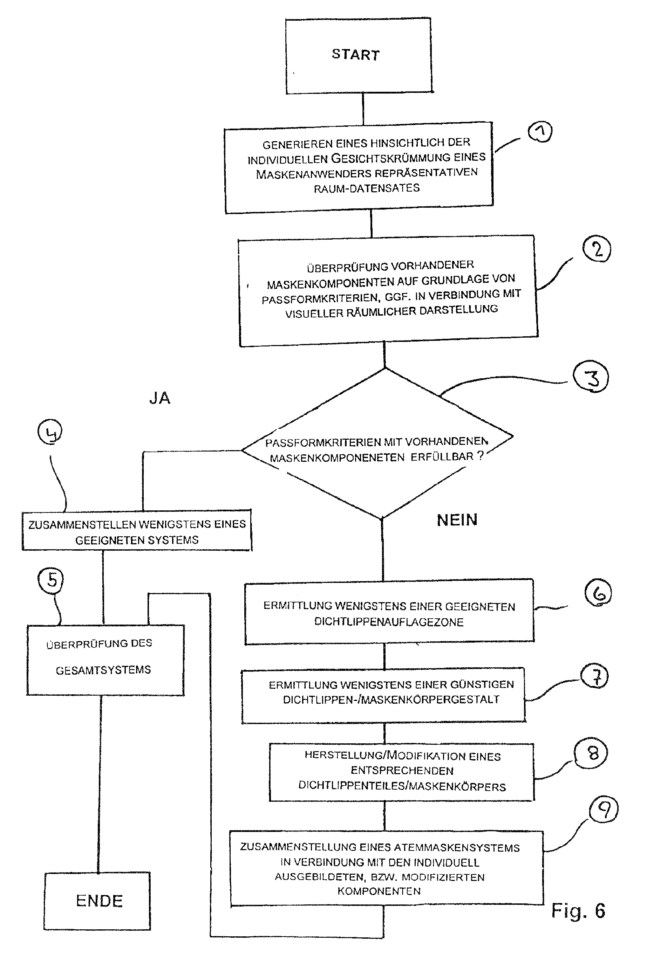

In Fig. 6 ist ein bevorzugter Ablauf des erfindungsgemäßen Verfahrens dargestellt. Im Rahmen des ersten Verfahrensschrittes 1 wird ein Raumdatensatz generiert, der die individuelle Gesichtskrümmung des Maskenanwenders im Umgebungsbereich der Nase sowie vorzugsweise auch im Stirnbereich beschreibt.

Im Rahmen des Verfahrensschrittes 2 wird auf Grundlage des generierten Raumdatensatzes überprüft, ob auf mit Komponenten vorhandener Maskensysteme (z.B. Dichtlippen, Stirnauflageelementen, CO₂-Austauschventilen, Maskenkörpern, Kopfbändern usw.) ein vorbestimmten Paßformkriterien Rechnung tragendes Maskensystem bereit gestellt werden kann. Diese Überprüfung kann ggf. durch entsprechende visuelle Unterstützung erfolgen. Hierzu wird beispielsweise auf Grundlage des eingelesenen Raumdatensatzes der relevante Gesichtsbereich des Patienten am Bildschirm eines PCs dargestellt, wobei es möglich ist, ebenfalls in Verbindung mit 3D-Koordinaten archivierte Maskensysteme zu "applizieren". Falls grundsätzlich mehrere Maskensysteme geeignet erscheinen können diese ermittelt und ggf. in Verbindung mit einer automatisch erstellten Beurteilungstabelle bewertet werden.

Im Rahmen des Verfahrensschrittes 3 wird dann überprüft, ob, bzw. inwieweit die Paßformkriterien tatsächlich erfüllt sind. Für den Fall, daß die Paßformkriterien erfüllt werden, wird wenigstens ein aus vorhandenen, kompatiblen Systemkomponenten gebildetes System zusammengestellt (Schritt 4). Dieses derart zusammengestellte System kann nochmals unter Zugrundelegung weiterer Kriterien überprüft und ggf. nochmals visuell dargestellt werden (Schritt 5). Die Auswahl, bzw. Zusammenstellung, eines geeigneten Atemmaskensystems auf Grundlage des die individuelle Gesichtskrümmung beschreibenden Raumdatensatzes ist in diesem Falle beendet.

Wird im Verfahrensschritt 3 festgestellt, daß mit den vorhandenen Maskenkomponenten voraussichtlich kein geeignetes Atemmaskensystem zusammengestellt werden kann, wird in Schritt 6 damit begonnen, einen vorteilhaften Verlauf für eine Dichtlippenaufiagezone im Gesicht des Maskenanwenders zu definieren.

Gem. Verfahrensschritt 7 wird für diese Dichtlippenauflagezone eine vorteilhafte Dichtlippen-/Maskenkörpergestalt ermittelt. Gem. Verfahrensschritt 8 wird nunmehr ein entsprechendes Dichtlippenteil, bzw. ein entsprechender Maskenkörper hergestellt. Dies kann beispielsweise erfolgen, in dem ein Stützstrukturteil, das das Dichtlippenteil in eine vorgegebene Konfiguration verformt, auf Grundlage von über den Raumdatensatz ermittelten Daten individuell verformt wird. Weiterhin ist es möglich, die Innenumfangskante des Dichtlippenteils definiert nachzuarbeiten. Der räumliche Verlauf der Dichtlippeninnenkante kann ebenfalls auf Grundlage entsprechender über den Raumdatensatz aufbereiteter Daten festgelegt werden.

Sobald die einer individuellen Anpassung unterliegenden Komponenten des Maskensystems vorliegen oder zumindest definiert sind, kann ebenfalls ein vollständiges Maskensystem zusammengestellt werden (Schritt 9). Dieses auf den jeweiligen Maskenanwender speziell abgestimmte Maskensystem kann dann wieder - wie in Verbindung mit dem Verfahrensschritt 5 bereits angegeben - ggf. unter Zugrundelegung weiterer Kriterien überprüft werden. Die Bereitstellung eines entsprechenden Maskensystems über den Weg der Sonderanfertigung oder individuellen Modifikation von Maskenkomponenten ist damit ebenfalls beendet.

Die für die Atemmaske oder für das gesamte Atemmaskensystem relevanten Geometriedaten des jeweiligen Maskenanwenders können in einer Datenbank gespeichert werden. Hierbei ist es möglich bei einer Erweiterung des Komponenten-Sortimentes zu überprüfen, ob auf Grundlage der neueren Komponenten den Anforderungen noch besser Rechnung getragen werden kann ohne daß der Maskenanwender vorab das Maskensystem erproben müßte. In weiterhin vorteilhafter Weise wird es auch möglich, überprüfbar festzustellen, ob tatsächlich Bedarf nach einer individuell angepaßten Maskenanordnung besteht oder ob die erwarteten Flächenpressungen derart niedrig sind, daß Beanstandungen hinsichtlich des Tragekomforts nicht zu erwarten sind.

## Patentansprüche

1. Verfahren zur Bereitstellung zur Ausbildung oder Auswahl einer Atemmaske für einen Maskenanwender geeigneter Angaben, wobei das Verfahren aufweist:
Erzeugen eines Raumdatensatzes durch Einlesen der individuellen räumlichen Gesichtskontur des Maskenanwenders zumindest im Nasen- und/oder Mundbereich,
wobei der Raumdatensatz durch einen optischen Abtastvorgang und/oder eine stereofotografische Messung erzeugt wird.

2. Verfahren nach Anspruch 1, **dadurch gekennzeichnet, dass** auf Grundlage des erfassten Raumdatensatzes ein Dichtlippenabschnitt der Atemmaske derart ausgebildet wird, dass dessen räumlicher Verlauf auf die individuelle Gesichtskrümmung im Dichtlippenauflagebereich abgestimmt ist.

3. Verfahren nach Anspruch 1, **dadurch gekennzeichnet, dass** auf Grundlage des erfassten Raumdatensatzes ein Dichtlippenteil und/oder ein Maskenbasiskörper und/oder ein Stirnauflageelement ausgewählt wird.

4. Verfahren nach wenigstens einem der Ansprüche 1 bis 3, **dadurch gekennzeichnet, dass** der Raumdatensatz in digitaler Form gespeichert wird, und dass auf Grundlage dieses Datensatzes ein geeigneter Verlauf der Gesichtsabdichtungszone ermittelt wird.

5. Verfahren nach wenigstens einem der Ansprüche 1 bis 4, **dadurch gekennzeichnet, dass** auf Grundlage des erfassten Raumdatensatzes wenigstens ein Maskenkörper aus einer Serie von Maskenkörpern derart ausgewählt wird, dass zwischen der Innenwandung des Maskenkörpers und dem Nasenbereich des Maskenanwenders ein hinreichender Zwischenraum verbleibt.

6. Verfahren nach wenigstens einem der Ansprüche 1 bis 5, **dadurch gekennzeichnet, dass** auf Grundlage des erfassten Raumdatensatzes wenigstens ein geeignetes Dichtlippenteil aus einer Serie von Dichtlippenteilen ausgewählt wird.

7. Verfahren nach wenigstens einem der Ansprüche 1 bis 6, **dadurch gekennzeichnet, dass** das Dichtlippenteil auf Grundlage des Raumdatensatzes modifiziert ausgebildet wird.

8. Verfahren nach wenigstens einem der Ansprüche 1 bis 7, **dadurch gekennzeichnet, dass** eine dem Dichtlippenteil zugeordnete Stützstruktur nach Maßgabe des Raumdatensatzes derart ausgebildet wird, dass die Stützstruktur das Dichtlippenteil in eine der individuellen Gesichtskrümmung im Bereich der Gesichtsauflagefläche Rechnung tragende Konfiguration zwängt.

9. Verfahren nach wenigstens einem der Ansprüche 1 bis 8, **dadurch gekennzeichnet**, **dadurch gekennzeichnet, dass** ein Verbindungsbereich zwischen dem Dichtlippenteil und dem Maskenkörper auf Grundlage des Raumdatensatzes ausgebildet wird.

10. Verfahren nach wenigstens einem der Ansprüche 1 bis 9, **dadurch gekennzeichnet, dass** der Raumdatensatz derart erzeugt wird, dass dieser die Krümmung des Gesichts zumindest abschnittsweise mit einer Auflösung von wenigstens 16 Messpunkten je cm² wiedergibt.

11. Verfahren nach wenigstens einem der Ansprüche 1 bis 10, **dadurch gekennzeichnet, dass** durch Interpolation der erfassten Koordinaten die Auflösung noch weiter verfeinert wird.

12. Verfahren nach wenigstens einem der Ansprüche 1 bis 11, **dadurch gekennzeichnet, dass** der Raumdatensatz durch einen laseroptischen Abtastvorgang erzeugt wird.

13. Verfahren nach wenigstens einem der Ansprüche 1 bis 12, **dadurch gekennzeichnet, dass** der Raumdatensatz dadurch erzeugt wird, dass mittels einer Digitalkamera das Gesicht des Maskenanwenders fotografiert wird, wobei auf das Gesicht aus einer vorzugsweise von der optischen Achse der Kamera abweichenden Richtung ein Muster projiziert wird.

14. Verfahren zur Bereitstellung einer Atemmaske, wobei das Verfahren zur Bereitstellung einer Atemmaske das Verfahren nach einem der vorangehenden Ansprüche aufweist.
